# EUROPÄISCHE PATENTSCHRIFT

(11) **EP 3 173 038 B1**
(45) Veröffentlichungstag und Bekanntmachung des Hinweises auf die Patenterteilung: **29.07.2020**
(21) Anmeldenummer: 16199196.3
(22) Anmeldetag: 16.11.2016
(51) Int. Cl.: A61B 17/32

(54) **SCHAFTINSTRUMENT**
SHAFT INSTRUMENT
INSTRUMENT À QUEUE

(30) Priorität: 19.11.2015 DE 102015222858
(43) Veröffentlichungstag der Anmeldung: 31.05.2017
(73) Patentinhaber: Richard Wolf GmbH, 75438 Knittlingen (DE)
(72) Erfinder: Frey, Sebastian, 68753 Waghäusel (DE); Körner, Eberhard, 75438 Knittlingen (DE)
(74) Vertreter: Patentanwälte Vollmann Hemmer Lindfeld Partnerschaft mbB

(56) Entgegenhaltungen:
- DE-A1-102006 000 399
- DE-U1-202009 007 979
- US-A- 5 851 212
- US-A1- 2012 083 770
- US-B1- 8 419 720

## Beschreibung

Die Erfindung betrifft ein Schaftinstrument, insbesondere ein medizinisch-endoskopisches Schaftinstrument.

Instrumente der in Rede stehenden Art werden standardmäßig im Bereich der minimal-invasiven Chirurgie eingesetzt. Dort wird ihr Schaft durch einen natürlich oder künstlich geschaffenen Zugangskanal in das Körperinnere des zu behandelnden Patienten eingeführt, so dass ein an dem distalen Ende des Schaftes angeordnetes Werkzeug in unmittelbarer Nähe des Behandlungsgegenstandes angeordnet werden kann, während sich proximalseitig des Schaftes angeordnete Einrichtungen zur Steuerung des Werkzeuges für den Operateur gut zugänglich außerhalb des Patienten befinden.

Neben solchen Schaftinstrumenten, bei denen das Werkzeug in direkter Längsverlängerung eines starren, geraden Schaftes angeordnet ist, ist aus US 5,851,212 A ein Instrument bekannt, bei dem ein distaler Endabschnitt des Schaftes quer zur Längsachse des Schaftes krümmbar ist. Während die erstgenannten Schaftinstrumente insbesondere dann an ihre Grenzen stoßen, wenn lediglich ein sehr enger Zugangskanal für das Instrument zur Verfügung steht und mit dem an dem distalen Ende des Schaftes angeordneten Werkzeug operative Manipulationen seitlich einer durch den Zugangskanal definierten Zugangsachse durchgeführt werden müssen, erscheint das aus US 5,851,212 A bekannte Instrument unter diesen Bedingungen aufgrund der Krümmbarkeit seines distalen Endabschnitts besser geeignet.

Die Dokumente DE 20 2009 007 979 U1 und US 8,419,720 B, offenbaren auch ähnliche bekannte Schaftinstrumente.

Bei diesem Instrument handelt es sich um einen sogenannten Shaver zum Abtragen von Körpergewebe. Zu diesem Zweck weist dieses Instrument ein rotierendes Schneidwerkzeug mit einer parallel zu seiner Rotationsachse ausgerichteten Schneide auf, welches in einem distalseitig des krümmbaren Schaftabschnitts angeordneten Instrumentenkopf angeordnet ist, der eine Gegenschneide zu der Schneide des Schneidwerkzeugs bildet.

Ein wesentlicher Bestandteil des krümmbaren Schaftabschnitts dieses Instruments ist eine quer zu ihrer Längsachse biegbare Schraubenfeder, die mit einem Ende an dem starr ausgebildeten proximalen Schaftabschnitt angeschweißt ist und mit dem anderen Ende an dem Instrumentenkopf angeschweißt ist. An der radialen Außenseite der Schraubenfeder ist ein Metallstreifen angeordnet, der sowohl mit der Schraubenfeder als auch mit dem proximalen Schaftabschnitt und dem Instrumentenkopf verschweißt ist. Ferner werden die Schraubenfeder und der Metallstreifen von einer Kunststoffhülle umgeben, die auf der Schraubenfeder aufgeschrumpft ist. Insofern ist insbesondere die Herstellung des krümmbaren Abschnitts des aus US 5,851,212 A bekannten Instruments vergleichsweise zeit- und kostenintensiv.

Vor diesem Hintergrund besteht die Aufgabe der Erfindung darin, ein Schaftinstrument der vorab beschriebenen Art mit einem relativ zu einem proximalen Schaftabschnitt seitlich auslenkbaren Schaftabschnitt zu schaffen, welches bei hoher Zuverlässigkeit einen geringeren Herstellungsaufwand verlangt.

Diese Aufgabe wird durch ein Schaftinstrument mit den in Anspruch 1 angegebenen Merkmalen gelöst, wobei sich vorteilhafte Weiterbildungen dieses Instruments aus den Unteransprüchen, der nachfolgenden Beschreibung sowie der Zeichnung ergeben. Hierbei können die in den Unteransprüchen angegebenen Merkmale vorteilhaft in der angegebenen Kombination aber auch, soweit technisch sinnvoll, für sich oder in anderer Kombination zur Ausgestaltung der Erfindung beitragen.

Das erfindungsgemäße Schaftinstrument ist bevorzugt ein medizinisch-endoskopisches Schaftinstrument, es kann sich bei ihm aber auch um ein im technischen Bereich eingesetztes Technoskop handeln. Das Schaftinstrument ist mit einem Hohlschaft ausgestattet, welcher zwischen zwei starr ausgebildeten Teilen einen quer zu seiner Längsausdehnung krümmbaren Teil aufweist. Somit ist der Hohlschaft mit einem starren, vorzugsweise gerade ausgerichteten proximalen Teil ausgestattet, an den sich ein Teil anschließt, welcher von einer Stellung, in der er in direkter Längsverlängerung des proximalen Teils des Hohlschafts ausgerichtet ist, in eine Richtung quer zu einer Längsachse des proximalen Teils des Hohlschafts gekrümmt werden kann. Auf den krümmbaren Teil folgt distalseitig ein zweiter, starr ausgebildeter Teil des Hohlschafts, der vorzugsweise einen distalen Instrumentenkopf des Schaftinstruments bildet, in welchem ein Werkzeug des Schaftinstruments gelagert ist.

Gemäß der Erfindung ist der krümmbare Teil des Hohlschafts mit zumindest einem der anderen Teile des Hohlschafts über eine Formschlussverbindung feststehend verbunden. Gegenüber der aus dem Stand der Technik vorbekannten stoffschlüssigen Verbindung des krümmbaren Teils des Hohlschafts mit dem starr ausgebildeten proximalen Teil des Hohlschafts und dem Instrumentenkopf hat die Formschlussverbindung des krümmbaren Teils mit einem oder vorzugsweise mit beiden der anderen Teile des Hohlschafts den Vorteil, dass die drei Teile des Hohlschafts, das heißt dessen beide starr ausgebildeten Teile und der dazwischen angeordnete krümmbare Teil aus unterschiedlichsten Materialien bestehen können, so dass beispielsweise der krümmbare Teil des Hohlschafts kostengünstig und im Hinblick auf seine Krümmbarkeit sinnvoll aus Kunststoff hergestellt sein kann, während es sich bei den beiden anderen Teilen des Hohlschafts um Metallteile handeln kann.

Zur Bildung der Formschlussverbindung zwischen dem krümmbaren Teil und zumindest einem der anderen Teile des Hohlschafts ist erfindungsgemäß vorgesehen, dass zwei an einer Umfangswandung eines der miteinander verbundenen Teile ausgebildete Vorsprünge, welche an der Umfangswandung einander gegenüberliegend angeordnet sind und in Richtung der Längsachse des Teils vorstehen, in zwei an der Umfangswandung des anderen Teils einer gegenüberliegend ausgebildete und die Umfangswandung vollständig durchbrechende Ausnehmungen formschlüssig eingreifen. Demzufolge stehen an der Umfangswandung eines ersten Teils des Hohlschafts zwei Vorsprünge in axialer Richtung des Teils und typischerweise in Richtung des damit zu verbindenden zweiten Teils vor, wobei diese beiden Vorsprünge einander gegenüberliegen und vorzugsweise einander diametral, das heißt auf einer die Mittelachse dieses Teils schneidenden Verbindungslinie gegenüberliegen. Korrespondierend hierzu sind an dem zweiten Teil des Hohlschafts, welcher mit dem ersten Teil verbunden ist, an einem dem ersten Teil zugewandten Ende an dessen Umfangswandung zwei mit den Vorsprüngen des ersten Teils komplementäre Ausnehmungen ausgebildet, die dessen Umfangswandung vollständig durchbrechen und einander ebenfalls gegenüberliegen bzw. einander vorzugsweise auf einer die Mittelachse dieses Teils schneidenden Verbindungslinie diametral gegenüberliegen, wobei die Vorsprünge des ersten Teils und die Ausnehmungen des zweiten Teils typischerweise Hinterschneidungen aufweisen, die einen Formschluss zwischen dem ersten und dem damit zu verbindenden zweiten Teil in Richtung der Längsausdehnung des Hohlschafts bewirken. Vorteilhaft ermöglicht die Anordnung der Vorsprünge an dem ersten Teil des Hohlschafts in Verbindung mit der Anordnung und Ausgestaltung des zweiten Teils des Hohlschafts eine schnelle und einfache Montage des ersten Teils an dem zweiten Teil, da hierzu der erste Teil ausgehend von einer Stellung, in der seine Vorsprünge in direkter, gerader Linie zu den Ausnehmungen des zweiten Teils außenseitig des zweiten Teils angeordnet sind, lediglich in gerader Richtung relativ zu dem zweiten Teil bewegt werden muss, bis jeder seiner Vorsprünge in einer Ausnehmung des zweiten Teils in Eingriff ist.

Da die beiden in der beschriebenen Weise verbundenen Teile des Hohlschafts durch den Eingriff der Vorsprünge des ersten Teils in die Ausnehmungen des zweiten Teils nur in axialer Richtung des Hohlschafts festgelegt sind, ist erfindungsgemäß ein Bauteil vorgesehen, welches die so formschlüssig miteinander verbundenen Teile umgibt. Hierdurch werden die beiden Teile des Hohlschafts in radialer Richtung an einer Bewegung relativ zu einander gehindert, so dass sie auch in radialer Richtung des Hohlschafts formschlüssig miteinander verbunden sind. Eine besonders einfache Montage dieses, die beiden miteinander verbundenen Teile umgebenden Bauteils ist dann möglich, wenn das Bauteil ringförmig ausgebildet ist und einen mit dem Außenquerschnitt der miteinander zu verbindenden Teile des Hohlschafts korrespondierenden Innenquerschnitt aufweist, so dass das Bauteil nur auf den Hohlschaft aufgesteckt werden muss, bis sich das Bauteil direkt außerhalb der Formschlussverbindung zwischen dem einen und dem anderen Teil des Hohlschafts befindet. Zweckmäßigerweise ist das Bauteil so dimensioniert, dass es den krümmbaren Teil des Hohlschafts lediglich in geringem Maße überdeckt, um auf diese Weise nicht dessen Krümmung zu be- bzw. zu verhindern.

Gemäß der Erfindung ist der distale Teil des Hohlschafts in der vorab beschriebenen Weise formschlüssig mit dem krümmbaren Teil des Hohlschafts verbunden. In diesem Fall ist eine Hülse vorgesehen, welche mit dem distalen Teil des Hohlschafts verbunden ist und die Formschlussverbindung zwischen dem distalen Teil und dem krümmbaren Teil radial umgibt. Zur formschlüssigen Festlegung des distalen Teils in Richtung der Längsachse des Hohlschafts sind hierbei bevorzugt die Vorsprünge an dem distalen Teil des Hohlschafts angeordnet, während die Ausnehmungen, in welche die Vorsprünge eingreifen, an dem krümmbaren Teil des Hohlschafts ausgebildet sind. In diesem Fall kann die Verbindung zwischen der Hülse und dem distalen Teil des Hohlschafts vorteilhaft dadurch realisiert werden, dass die an dem distalen Teil ausgebildeten Vorsprünge nicht nur in dessen axialer Richtung, sondern auch in dessen radialer Richtung vorstehen und in an der Hülse ausgebildete, mit den Vorsprüngen korrespondieren Durchbrechungen eingreifen. Hierdurch ist die Hülse gegenüber dem distalen Teil des Hohlschafts und gegenüber dessen krümmbaren Teil unbewegbar. Zusätzlich oder alternativ zu dieser Festlegung der Hülse können der distale Teil und die Hülse auch stoffschlüssig, beispielsweise über eine Laserschweißung, miteinander verbunden sein.

Nach einer weiteren, bevorzugten Ausgestaltung des erfindungsgemäßen Schaftinstruments ist der krümmbare Teil des Hohlschafts in der vorab beschriebenen Weise formschlüssig mit dem proximalen Teil des Hohlschafts verbunden. Hierbei umgibt ein Außenrohr die Formschlussverbindung zwischen dem krümmbaren und dem proximalen Teil des Hohlschafts radial, wobei der Hohlschaft relativ zu dem Außenrohr axial bewegbar ist. Das Außenrohr erstreckt sich zweckmäßigerweise bis zu einer an dem Schaftinstrument proximal angeordneten Handhabe, wobei der Hohlschaft in der Handhabe proximalseitig aus dem Außenrohr herausragt und in diesem Bereich mit einer Steuereinrichtung wirkungsverbunden ist, mit welcher er relativ zu dem Außenrohr axial bewegbar ist. Bezüglich dieser Bewegbarkeit des Hohlschafts, auf deren Bedeutung nachfolgend noch eingehender eingegangen wird, versteht es sich von selbst, dass der Bewegungsweg des Hohlschafts relativ zu dem Außenrohr derart begrenzt ist, dass das Außenrohr die Formschlussverbindung zwischen dem krümmbaren und dem proximalen Teil des Hohlschafts immer umgibt.

Zur Krümmung des krümmbaren Teils des Hohlschafts ist zweckmäßigerweise ein Zugelement vorgesehen, welches distalseitig des krümmbaren Teils oder im Bereich des Endes des krümmbaren Teils an dem Hohlschaft befestigt ist und bei einer Kraftbeaufschlagung eine seitliche Auslenkung des krümmbaren Teils bewirkt. Erfindungsgemäß bevorzugt ist eine Ausgestaltung, bei welcher die mit dem distalen Teil des Hohlschafts verbundene Hülse mit einem solchen Zugelement zur Steuerung der Krümmung des krümmbaren Teils des Hohlschafts verbunden ist.

Insbesondere in Weiterbildung dieser Ausgestaltung ist es von besonderem Vorteil, wenn, wie es weiter bevorzugt vorgesehen ist, das Zugelement mit dem die Formschlussverbindung zwischen dem krümmbaren und dem proximalen Teil des Hohlschafts radial umgebenden Außenrohr verbunden ist. In diesem Fall ist das Zugelement somit nicht nur mit dem Teil der Formschlussverbindung zwischen dem distalen und dem krümmbaren Teil des Hohlschafts bildenden Hülse, sondern auch mit dem Außenrohr verbunden. Wird bei dieser Ausgestaltung auf den Hohlschaft eine in dessen distale Richtung wirkende Bewegungskraft ausgeübt, ist eine damit verbundene Bewegung des Hohlschafts nur unter gleichzeitiger Krümmung des krümmbaren Teils des Hohlschafts möglich, der krümmbare Teil wird somit in Abhängigkeit des Bewegungswegs des Hohlschafts seitlich ausgelenkt.

Vor allem im Hinblick auf eine möglichst geringe Bauteilanzahl des Schaftinstruments ist bevorzugt vorgesehen, dass die Hülse und das Zugelement von einem gemeinsamen Bauteil gebildet werden. Zur Herstellung dieses Bauteils wird zweckmäßigerweise ein dünnwandiges Rohr verwendet, dessen Wandung abgesehen von einem die Hülse bildenden Endabschnitt in einem Winkelbereich von beispielsweise 340° oder mehr entfernt wird, so dass an dem die Hülse bildenden Endabschnitt ein in axialer Richtung vorstehender schmaler Materialstreifen verbleibt, welcher das Zugelement bildet.

Der krümmbare Teil des Hohlschafts wird bevorzugt von einem Rohr gebildet. Um eine seitliche Auslenkung des krümmbaren Teils zu ermöglichen, ist dieser vorteilhaft aus einem elastischen Material ausgebildet, wobei er über seine Länge verteilt mehrere quer zu seiner Längsausdehnung verlaufende Einschnitte aufweist. Hierbei sind mehrere der Einschnitte zweckmäßigerweise an der Außenseite des krümmbaren Teils des Hohlschafts ausgebildet, außenseitig derer das Zugelement zur Steuerung der seitlichen Auslenkung des krümmbaren Teils angeordnet ist und dessen Richtung der krümmbare Teil auslenkbar ist. Damit eine Krümmung dieses Teils des Hohlschafts nicht mit einer Materialverdrängung in das Innenlumen dieses Teils des Hohlschafts einhergeht, sind die Einschnitte günstigerweise keilförmig erweitert ausgebildet.

Zu diesem Zweck ist auch vorteilhaft vorgesehen, dass direkt aufeinander folgende Einschnitte an einander direkt gegenüberliegenden Wandungsbereichen des krümmbaren Teils des Hohlschafts ausgebildet sind. Demzufolge folgt einem Einschnitt, welcher an einem Wandungsbereich ausgebildet ist, der dem außenseitig des krümmbaren Teils des Hohlschafts angeordneten Zugelement zugewandt ist, ein Einschnitt, der an einem von dem Zugelement abgewandten Wandungsbereich ausgebildet ist und diesem wieder ein Einschnitt, welcher an dem Wandungsbereich ausgebildet ist, der dem Zugelement zugewandt ist. Typischerweise können diesem letztgenannten Einschnitt weitere Einschnitte folgen, die in der beschriebenen Weise an dem krümmbaren Teil des Hohlschafts angeordnet sind.

Für eine definierte seitliche Auslenkung des krümmbaren Teils des Hohlschafts ist es von maßgeblicher Bedeutung, dass das Zugelement immer parallel zu dem krümmbaren Teil des Hohlschafts ausgerichtet ist. Vor allem hierauf zielt eine weitere vorteilhafte Weiterbildung des erfindungsgemäßen Instruments ab, nach der an dem krümmbaren Teil des Hohlschafts außenseitig mehrere Erhebungen ausgebildet sind, durch welche das Zugelement geführt ist. In Folge der Führung des Zugelementes durch die an dem krümmbaren Teil des Hohlschafts ausgebildeten Erhebungen, in denen das Zugelement in seiner axialen Richtung frei bewegbar ist, liegt das Zugelement unabhängig von einer Krümmung des krümmbaren Teils immer eng an letztgenanntem Teil an.

Generell ist die Art eines in Verbindung mit dem erfindungsgemäßen Schaftinstrument verwendeten Werkzeugs und damit einhergehend der Verwendungszweck des erfindungsgemäßen Schaftinstruments weitestgehend beliebig. Aufgrund des krümmbaren Teils seines Hohlschafts und der damit verbundenen seitlichen Auslenkbarkeit eines distalseitig des krümmbaren Teils angeordneten Werkzeuges eignet sich dieses Schaftinstrument aber in besonderem Maße zum Abtrag von Körpergewebe und insbesondere zum Gewebeabtrag bei Bandscheibenoperationen, bei denen das Werkzeug besonders genau zu dem abzutragenden Gewebe ausgerichtet werden kann, so dass die Gefahr einer Schädigung von anderem Gewebe erheblich verringert wird. Insofern handelt es sich bei dem erfindungsgemäßen Instrument vorzugsweise um ein Schneidinstrument, bei welchem in dem distalen Teil des Hohlschafts ein dort zumindest teilweise freiliegendes Schneidwerkzeug angeordnet ist, welches von einer durch den Hohlschaft geführten Antriebswelle rotierend angetrieben wird.

Nachfolgend ist die Erfindung anhand eines in der Zeichnung dargestellten Ausführungsbeispiels näher erläutert. In der Zeichnung zeigt schematisch vereinfacht und in unterschiedlichen Maßstäben:

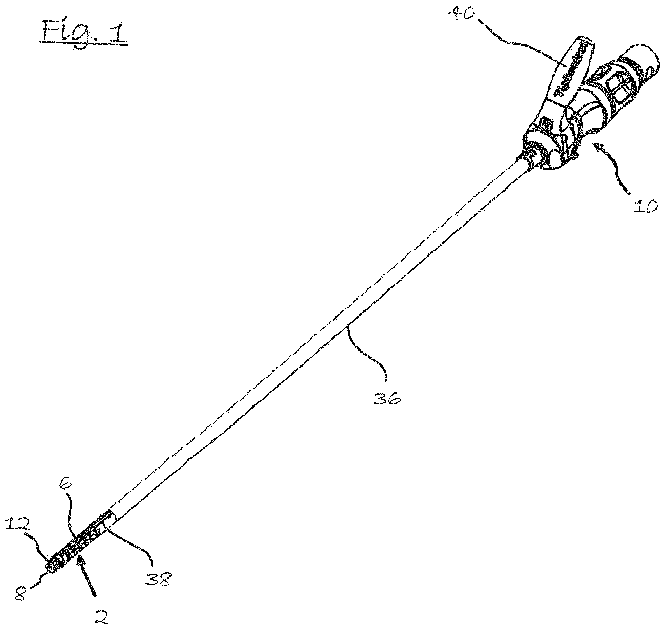

- Fig. 1: in perspektivischer Darstellung ein medizinischendoskopisches Schaftinstrument,
- Fig. 2: in perspektivischer Darstellung einen distalen Endbereich des Schaftinstruments nach Fig. 1.,
- Fig. 3: in perspektivischer Explosionsdarstellung einen proximalen und einen sich daran anschließenden krümmbaren Teil eines Schaftes des Schaftinstruments nach Fig. 1,
- Fig.4: die Darstellung nach Fig. 3 im zusammengefügten Zustand,
- Fig. 5: in perspektivischer Explosionsdarstellung den krümmbaren Teil des Schaftes und einen sich daran anschließenden distalen Teil des Schaftes des Schaftinstruments nach Fig. 1,
- Fig. 6: die Darstellung nach Fig. 5 im zusammengefügten Zustand,

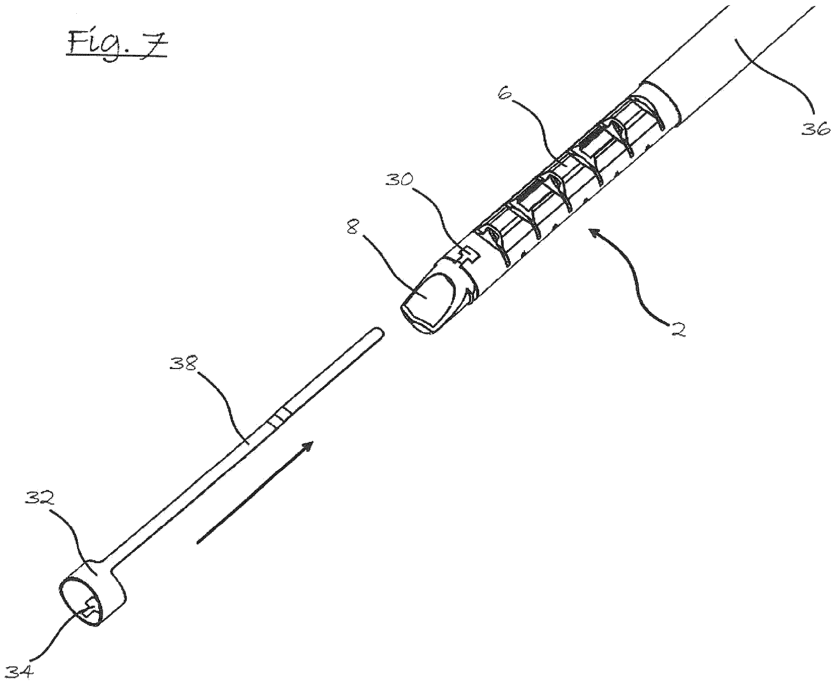

- Fig. 7: in perspektivischer Explosionsdarstellung einen distalen Endabschnitt des Schaftes und ein Zugelement zum Auslenken des krümmbaren Teils des Schaftes und

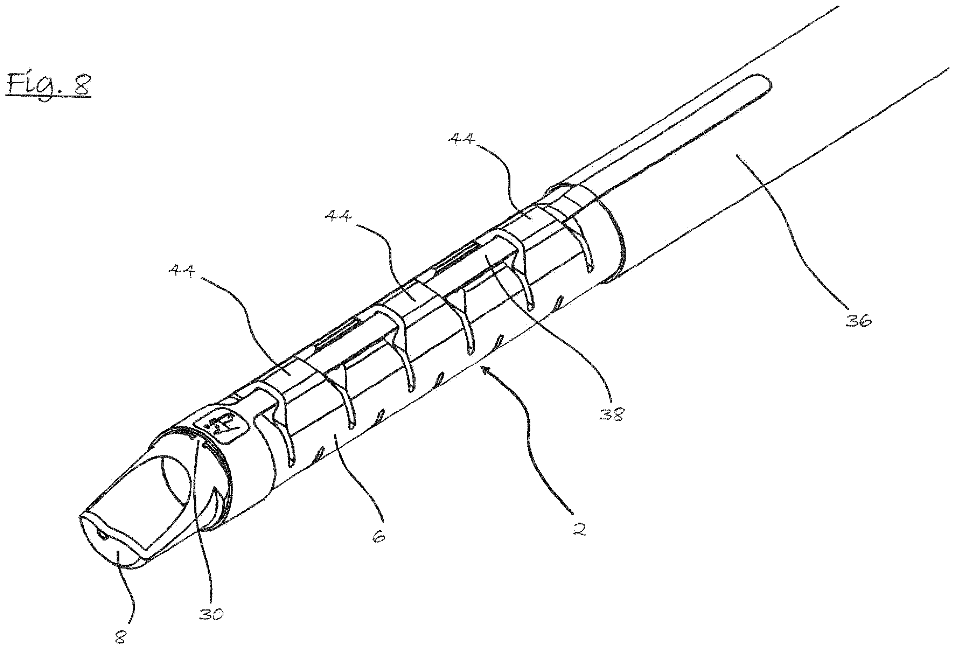

- Fig. 8: die Darstellung nach Fig. 7 im zusammengefügten Zustand.

Bei dem in der Zeichnung dargestellten Schaftinstrument handelt es sich um ein Schneidinstrument zum Abtragen von Körpergewebe. Dieses Schaftinstrument weist einen länglichen Hohlschaft 2 auf, der dreiteilig ausgebildet ist und einen gerade ausgerichteten proximalen Teil 4, einen sich daran distalseitig anschließenden krümmbaren Teil 6 und einen das distale Ende des Schaftinstruments bildenden Teil 8 aufweist. Proximalseitig des Hohlschafts 2 ist eine Handhabe angeordnet, von der in der Zeichnung aus Übersichtlichkeitsgründen nur ein in ein Gehäuse der Handhabe eingreifender Einsatz 10 dargestellt ist, der eine Steuereinrichtung 40 zur Steuerung einer seitlichen Auslenkung des krümmbaren Teils 6 beinhaltet.

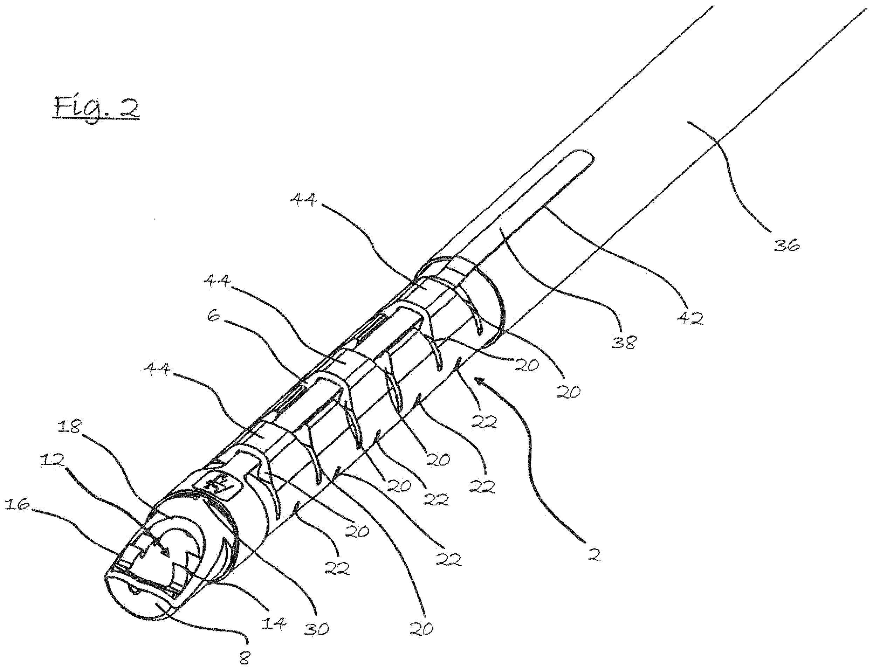

Der Teil 8 bildet einen Instrumentenkopf des Schaftinstruments, in dem ein Schneidwerkzeug 12 drehbar gelagert ist, welches über eine durch den Hohlschaft 2 geführte, in der Zeichnung nicht dargestellte Antriebwelle, mit einem in der proximalseitg des Hohlschafts 2 angeordneten Handhabe angeordneten und ebenfalls nicht dargestellten Antriebsmotor wirkungsverbunden ist. Das Schneidwerkzeug 12 weist einen im Wesentlichen zylindrischen Grundkörper auf, an dessen Umfangsseite ein Fenster ausgebildet ist, wobei zwei in axialer Richtung des Schneidwerkzeugs 12 verlaufende Längsseiten des Fensters zwei Schneiden 14 und 16 bilden. Die Schneiden 14 und 16 liegen an einem an dem den Instrumentenkopf bildenden Teil 8 ausgebildeten Fenster 18 frei (siehe insbesondere Fig. 2).

Der krümmbare Teil 6 des Hohlschafts 2 ist quer zur Längsausdehnung des proximalen Teils 4 des Hohlschafts 2, welcher, wie der Teil 8 des Hohlschafts 2, starr ausgebildet ist, seitlich auslenkbar. Zu diesem Zweck ist der im Wesentlichen rohrförmig ausgebildete Teil 6 des Hohlschafts 2 aus einem elastischen Kunststoff ausgebildet und weist über seine Länge verteilt mehrere Einschnitte 20 und 22 auf. Jeder dieser Einschnitte 20 und 22 ist quer zur Längsausdehnung des Teils 6 ausgerichtet und nach außen keilförmig erweitert. Hierbei sind die Einschnitte 20 in Längsrichtung des Teils 6 voneinander beabstandet an einem gemeinsamen Wandungsbereich des Teils 6 angeordnet, während die in Längsrichtung des Teils 6 zwischen den Einschnitten 20 ausgebildeten Einschnitte 22 an einem dem erstgenannten Wandungsbereich direkt gegenüberliegenden Wandungsbereich des Teils 6 ausgebildet sind (Fig. 2).

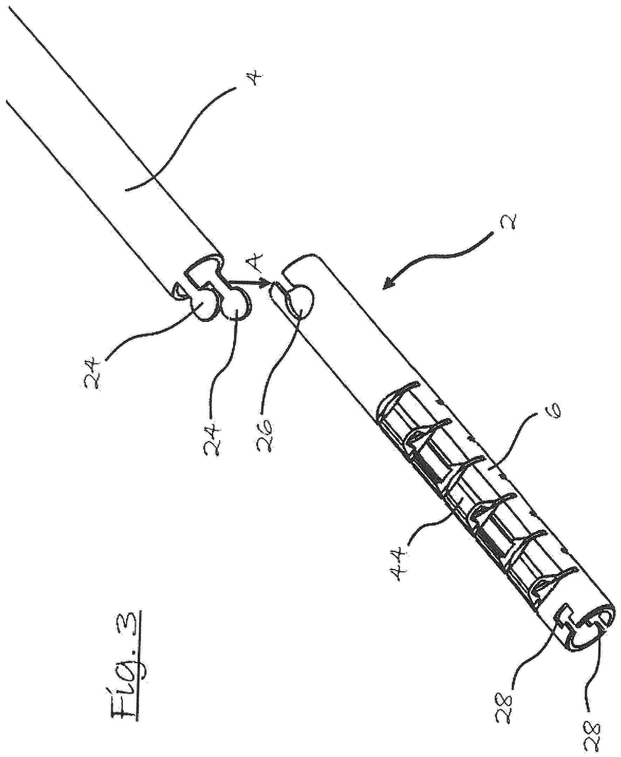

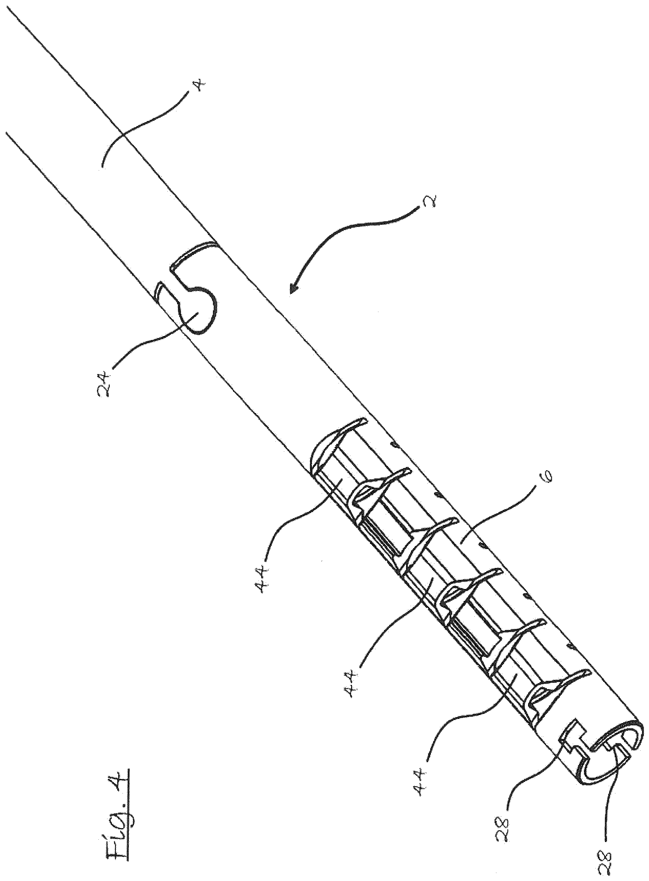

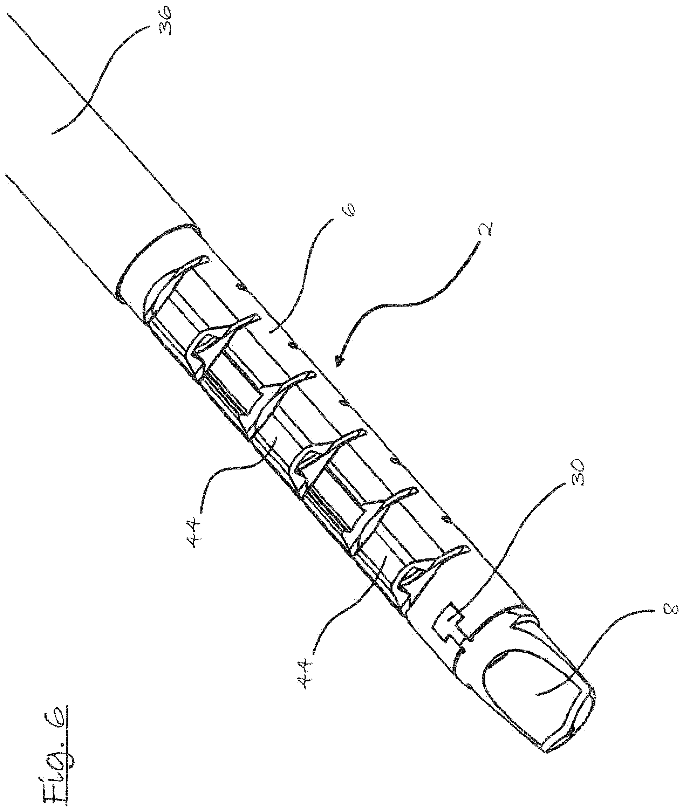

Aus den Fig. 3 - 6 wird deutlich, dass der zwischen den Teilen 4 und 8 liegende Teil 6 des Hohlschafts 2 mit den Teilen 4 und 8 formschlüssig verbunden ist.

Zur Verbindung des krümmbaren Teils 6 mit dem proximalen Teil 8 des Hohlschafts 2 sind an dem distalen Ende des proximalen Teils 4 des Hohlschafts 2 an der Umfangswandung des Teils 4 zwei im Wesentlichen T-förmige Vorsprünge 24 ausgebildet. Diese beiden Vorsprünge 24 sind an der Umfangswandung des Teils 4 einander diametral gegenüberliegend angeordnet und weisen in distale Richtung. Korrespondierend zu den an dem Teil 4 des Hohlschafts 2 ausgebildeten Vorsprüngen 24 sind an dem krümmbaren Teil 6 des Hohlschafts 2 ausgehend von dessen proximalen Ende zwei die Umfangswandung des Teils 6 vollständig durchbrechende T-förmige Ausnehmungen 26 ausgebildet, welche einander ebenfalls diametral gegenüberliegen. Wie aus Fig. 3 deutlich wird, wird zum Zusammenfügen der Teile 4 und 6 des Hohlschafts 2 der Teil 4 gegenüber dem Teil 6 so positioniert, dass seine Vorsprünge 24 in gerader Linie außenseitig der an dem krümmbaren Teil 6 ausgebildeten Ausnehmungen 26 angeordnet sind, wobei die Vorsprünge 24 des Teils 4 durch eine Bewegung des Teils 4 in eine Richtung A in einfacher Weise zum Eingriff in die Ausnehmungen 26 des Teils 6 des Hohlschafts gebracht werden können, wodurch der Teil 6 in Längsrichtung des Hohlschafts 2 formschlüssig an dem Teil 4 des Hohlschafts 2 festgelegt wird.

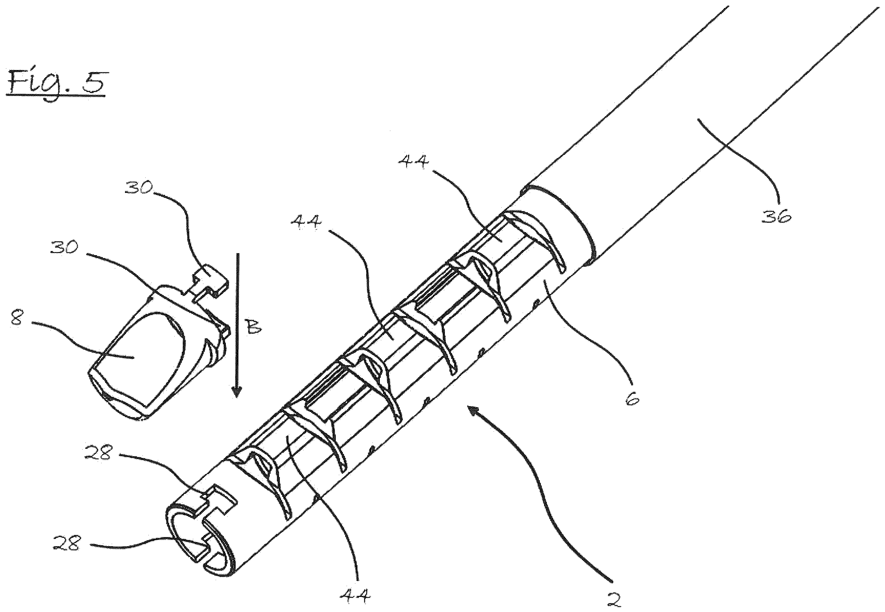

Ausgehend von dem distalen Ende des Teils 6 des Hohlschafts 2 sind an der Umfangswandung des Teils 6 zwei weitere T-förmige Ausnehmungen 28 ausgebildet, die einander in einer Ebene normal zu der Anordnung der Ausnehmungen 26 diametral gegenüberliegen. Korrespondierend zu diesen Ausnehmungen 28 sind an der Umfangswandung des distalen Teils 8 des Hohlschafts 2 zwei T-förmige Vorsprünge 30 ausgebildet, die an dem Teil 8 in proximaler Richtung vorstehen und einander an der Umfangswandung des Teils 8 diametral gegenüberliegend angeordnet sind. Aus Fig. 5 ist erkennbar, dass zum Zusammenfügen der Teile 6 und 8 des Hohlschafts 2 der Teil 8 so gegenüber dem Teil 6 positioniert wird, dass seine Vorsprünge 30 in gerader Linie außenseitig der an dem krümmbaren Teil 6 ausgebildeten Ausnehmungen 30 angeordnet sind, wobei die Vorsprünge 30 des Teils 8 durch eine Bewegung des Teils 8 in eine Richtung B in Eingriff mit den Ausnehmungen 28 des Teils 6 des Hohlschafts 2 gebracht werden können, wodurch der Teil 8 in Längsrichtung des Hohlschafts 2 formschlüssig an dem Teil 6 des Hohlschafts 2 festgelegt wird.

Um auch eine formschlüssige Festlegung des Teils 8 des Hohlschafts 2 an dessen Teil 6 in radialer Richtung des Hohlschafts 2 zu schaffen, ist eine zylindrische Hülse 32 vorgesehen. Diese Hülse 32 weist einen Innenquerschnitt auf, der mit dem Außenquerschnitt der Teile 6 und 8 korrespondiert. An einem Ende der Hülse 32 steht ein schmaler, länglicher Materialstreifen in axialer Richtung der Hülse vor, auf dessen Bedeutung nachfolgend noch ausführlicher eingegangen wird. Die Hülse 32 wird mit dem in proximale Richtung weisenden Materialstreifen so auf den Hohlschaft 2 aufgesetzt, dass sie den Teil 6 und den Teil 8 im Bereich deren Formschlussverbindung überdeckt. Hierbei greifen die an dem Teil 8 des Hohlschafts 2 ausgebildeten Vorsprünge 30, welche nicht nur in axialer Richtung sondern auch in radialer Richtung des Teils 8 vorstehen, in korrespondierende Ausnehmungen 34 der Hülse 32, welche die Hülse 32 vollständig durchbrechen. Durch diesen Eingriff der Vorsprünge 32 in die Ausnehmungen 34 ist die Hülse 32 fest mit dem Teil 8 des Hohlschafts 2 verbunden und somit nicht gegenüber der Formschlussverbindung zwischen dem Teil 6 und dem Teil 8 des Hohlschafts 2 bewegbar. Zusätzlich ist die Hülse 32 über Laserschweißpunkte mit dem Teil 8 des Hohlschafts 2 stoffschlüssig verbunden.

Zur Schaffung einer formschlüssigen Festlegung des krümmbaren Teils 6 des Hohlschafts an dessen proximalen Teil 4 in radialer Richtung des Hohlschafts 2 ist ein Außenrohr 36 vorgesehen. Dieses Außenrohr 36, das sich in proximale Richtung bis zu dem Einsatz 10 der Handhabe erstreckt und dort mit dem Einsatz 10 fest verbunden ist, überdeckt die von den Vorsprüngen 24 am Teil 4 und den Ausnehmungen 26 am Teil 6 des Hohlschafts 2 gebildete Formschlussverbindung und bildet somit auch einen Formschluss in radialer Richtung des Hohlschafts 2.

Gegenüber dem Außenrohr 36 ist der Hohlschaft 2 in axialer Richtung verschiebbar. Hierzu ist der Hohlschaft 2 mit einem an dem Einsatz 10 der Handhabe schwenkbar angeordneten Steuerhebel 40 bewegungsgekoppelt. Die in der Zeichnung nicht dargestellte Bewegungskopplung des Hohlschafts 2 mit dem Steuerhebel 40 ist derart, dass dann, wenn der Steuerhebel 40 in Richtung einer Mittelachse des Hohlschafts 2 verschwenkt wird, der Hohlschaft 2 relativ zu dem Außenrohr in distale Richtung bewegt wird.

Der an der Hülse 32 ausgebildete Materialstreifen bildet ein Zugelement 38 zur Steuerung einer seitlichen Auslenkung des krümmbaren Teils 6 des Hohlschafts 2. Das Zugelement 38 ist derart lang ausgebildet, dass es sich über das distale Ende des Außenrohrs 36 erstreckt und in diesem Bereich über eine Laserschweißnaht 42 fest mit dem Außenrohr 36 verbunden ist. Hierbei ist das Zugelement 36 durch mehrere radial außenseitig an dem krümmbaren Teil 6 des Hohlschafts 2 zwischen benachbarten Einschnitten 20 ausgebildete Erhebungen 44 frei beweglich hindurch geführt, so dass sich das Zugelement 38 immer unmittelbar außenseitig des Teils 6 des Hohlschafts 2 befindet. Zur seitlichen Auslenkung des krümmbaren Teils 6 des Hohlschafts 2 wird über den Steuerhebel 40 auf den Hohlschaft 2 eine in distale Richtung wirkende Bewegungskraft ausgeübt, so dass sich die Teile 4 und 6 des Hohlschafts 2 in distaler Richtung bewegen. Da aber der Teil 8 des Hohlschafts über das an der Hülse 32 ausgebildete Zugelement 38 fest mit dem Außenrohr 36 verbunden ist, wird hierdurch eine seitliche Auslenkung des Teils 6 des Hohlschafts in Richtung des Zugelements 38 quer zur Längsausdehnung des Hohlschafts 2 bewirkt. Sobald der Steuerhebel 40 losgelassen wird, bewirken die dem elastisch ausgebildeten Teil 6 des Hohlschafts 2 und dem Zugelement 38 innewohnenden Federeigenschaften, dass sich der Hohlschaft 2 in proximale Richtung zurückbewegt und der krümmbare Teil 6 des Hohlschafts wieder eine Form annimmt, in der er in gerader Längsverlängerung des proximalen Teil 4 des Hohlschafts 2 ausgerichtet ist.

### Bezugszeichenliste

- 2 -: Hohlschaft
- 4 -: Teil
- 6 -: Teil
- 8 -: Teil
- 10 -: Einsatz
- 12 -: Schneidwerkzeug
- 14 -: Schneide
- 16 -: Schneide
- 18 -: Fenster
- 20 -: Einschnitt
- 22 -: Einschnitt
- 24 -: Vorsprung
- 26 -: Ausnehmung
- 28 -: Ausnehmung
- 30: Vorsprung
- 32: Hülse
- 34: Ausnehmung
- 36: Außenrohr
- 38: Zugelement
- 40: Steuerhebel
- 42: Laserschweißnaht
- 44: Erhebung

- A -: Richtung
- B -: Richtung

## Patentansprüche

1. Schaftinstrument, insbesondere medizinisch-endoskopisches Schaftinstrument, mit einem Hohlschaft (2), welcher zwischen zwei starr ausgebildeten Teilen (4, 8) einen quer zu seiner Längsausdehnung krümmbaren Teil (6) aufweist, der mit zumindest einem der starr ausgebildeten anderen Teile (4, 8) derart feststehend verbunden ist, dass zwei an einer Umfangswandung eines der miteinander verbundenen Teile (4, 8) ausgebildete Vorsprünge (24, 30), welche an der Umfangswandung einander gegenüberliegend angeordnet sind und in Richtung der Längsachse des Teils (4, 8) vorstehen, in zwei an der Umfangswandung des anderen Teils (6) einander gegenüberliegend ausgebildete und die Umfangswandung vollständig durchbrechende Ausnehmungen (26, 28) formschlüssig eingreifen, wobei die miteinander formschlüssig verbundenen Teile (4, 6, 8) des Hohlschafts (2) im Bereich ihrer formschlüssigen Verbindung außenumfänglich von einem Bauteil umgeben sind, wobei der distale Teil des Hohlschafts (8) formschlüssig mit dessen krümmbaren Teil (6) verbunden ist, **dadurch gekennzeichnet, dass** eine mit dem distalen Teil (6) verbundene Hülse (32) den distalen Teil (8) und den krümmbaren Teil (6) des Hohlschafts (2) im Bereich deren Formschlussverbindung radial umgibt und die mit dem distalen Teil (8) des Hohlschafts (2) verbundene Hülse (36) mit einem Zugelement (38) zur Steuerung der Krümmung des krümmbaren Teils (6) des Hohlschafts (2) verbunden ist.

2. Schaftinstrument nach Anspruch 1, **dadurch gekennzeichnet, dass** der krümmbare Teil (6) des Hohlschafts (2) formschlüssig mit dem proximalen Teil (4) des Hohlschafts (2) verbunden ist und ein sich zumindest bis zum proximalen Ende des Hohlschafts (2) erstreckendes Außenrohr (36) die Formschlussverbindung zwischen dem krümmbaren (6) und dem proximalen Teil (4) des Hohlschafts (2) radial umgibt, wobei der Hohlschaft (2) relativ zu dem Außenrohr (36) axial bewegbar ist.

3. Schaftinstrument nach Anspruch 1 oder 2, **dadurch gekennzeichnet, dass** das Zugelement (38) mit dem die Formschlussverbindung zwischen dem krümmbaren (6) und dem proximalen Teil (4) des Hohlschafts (2) radial umgebenden Außenrohr (36) verbunden ist.

4. Schaftinstrument nach einem der vorhergehenden Ansprüche , **dadurch gekennzeichnet, dass** die Hülse (32) und das Zugelement (38) von einem gemeinsamen Bauteil gebildet werden.

5. Schaftinstrument nach einem der vorangehenden Ansprüche, **dadurch gekennzeichnet, dass** der krümmbare Teil (6) des Hohlschafts (2) aus einem elastischen Material ausgebildet ist und über seine Länge verteilt mehrere quer zu seiner Längsausdehnung verlaufende Einschnitte (20, 22) aufweist.

6. Schaftinstrument nach Anspruch 5, **dadurch gekennzeichnet, dass** direkt aufeinander folgende Einschnitte (20, 22) an einander direkt gegenüberliegenden Wandungsbereichen des krümmbaren Teils (6) des Hohlschafts (2) ausgebildet sind.

7. Schaftinstrument nach einem der vorangehenden Ansprüche , **dadurch gekennzeichnet, dass** an dem krümmbaren Teil (6) des Hohlschafts (2) außenseitig mehrere Erhebungen (44) ausgebildet sind, durch welche das Zugelement (38) geführt ist.

8. Schaftinstrument nach einem der vorangehenden Ansprüche, **dadurch gekennzeichnet, dass** in dem distalen Teil (8) des Hohlschafts (2) ein dort zumindest teilweise freiliegendes Schneidwerkzeug (12) angeordnet ist, welches von einer durch den Hohlschaft (2) geführten Antriebswelle angetrieben ist.

## Claims

1. A shank instrument, in particular a medical-endoscopic shank instrument, with a hollow shank (2) which between two rigidly designed parts (4, 8) comprises a part (6) which is bendable transversely to its longitudinal extension and which is stationarily connected to at least one of the other rigidly formed other parts (4, 8) in a manner such that two projections (24, 30) which are formed on a peripheral wall of one of the parts (4, 8) connected to one another, and are arranged lying opposite one another on the peripheral wall and project in the direction of the longitudinal axis of the part (4, 8) positively engage into two recesses (26, 28) which are formed lying opposite one another on the peripheral wall of the other part (6) and which completely break through the peripheral wall, wherein the parts (4, 6, 8) of the hollow shank (2) which are positively connected to one another, in the region of their positive connection are surrounded at the outer periphery by a component, wherein the distal part of the hollow shank (8) is positively connected to its bendable part (6), **characterised in that** a sleeve (32) which is connected to the distal part (6) radially surrounds the distal part (8) and the bendable part (6) of the hollow shank (2) in the region of their positive connection and the sleeve (36) which is connected to the distal part (8) of the hollow shank (2) is connected to a pull element (38) for the control of the bending of the bendable part (6) of the hollow shank (2).

2. A shank instrument according to claims 1, **characterised in that** the bendable part (6) of the hollow shank (2) is positively connected to the proximal part (4) of the hollow shank (2), and an outer tube (36) which extends at least up to the proximal end of the hollow shank (2) radially surrounds the positive connection between the bendable (6) and the proximal part (4) of the hollow shank (2), wherein the hollow shank (2) is axially movable relative to the outer tube (36).

3. A shank instrument according to claim 1 or 2, **characterised in that** the pull element (38) is connected to the outer tube (36) which radially surrounds the positive connection between the bendable (6) and the proximal part (4) of the hollow shank (2).

4. A shank instrument according to one of the preceding claims, **characterised in that** the sleeve (32) and the pull element (38) are formed by a common component.

5. A shank instrument according to one of the preceding claims, **characterised in that** the bendable part (6) of the hollow shank (2) is formed from an elastic material and comprises several incisions (20, 22) which run transversely to its longitudinal extension in a manner distributed over its length.

6. A shank instrument according to claim 5, **characterised in that** directly consecutive incisions (20, 22) are formed on wall regions of the bendable part (6) of the hollow shank (2), said wall regions lying directly opposite one another.

7. A shank instrument according to one of the preceding claims, **characterised in that** several prominences (44), through which the pull element (38) is led, are formed on the bendable part (6) of the hollow shank (2) at the outer side.

8. A shank instrument according to one of the preceding claims, **characterised in that** a cutting tool (12) which is at least partly exposed in the distal part (8) of the hollow shank (2) and which is driven by a drive shaft led through the hollow shaft (2) is arranged in the distal part (8) of the hollow shank (2).

## Revendications

1. Instrument à queue, notamment instrument à queue endoscopique médical, comprenant une queue creuse (2) qui comprend, entre deux parties rigides (4, 8), une partie (6) adaptée pour pouvoir être cintrée dans une direction transversale à son étendue longitudinale et qui est reliée rigidement à au moins une des deux autres parties (4, 8), formées de manière rigide, de façon que deux saillies (24, 30) formées sur une paroi périphérique de l'une des deux parties (4, 8) reliées l'une à l'autre et qui sont disposées l'une opposée à l'autre sur la paroi périphérique et qui sont en saillie dans la direction de l'axe longitudinal de la partie (4, 8), s'engagent, par complémentarité de forme, dans deux évidements (26, 28) formés sur la paroi périphérique de l'autre partie (6), l'un opposé à l'autre et traversant la paroi périphérique entièrement, les parties (4, 6, 8) de la queue creuse (2) reliées les unes aux autres par complémentarité de forme étant entourées à leur pourtour extérieur, dans la zone de leur liaison par complémentarité de forme, d'un composant, la partie distale (8) de la queue creuse étant reliée par complémentarité de forme à sa partie pouvant être courbée (6), **caractérisé en ce qu'**un manchon (32) relié à la partie distale (6) entoure radialement la partie distale (8) et la partie pouvant être courbée (6) de la queue creuse (2) dans la zone de leur liaison par complémentarité de forme et **en ce que** le manchon (32) relié à la partie distale (8) de la queue creuse (2) est relié à un élément de traction (38) pour commander le cintrage de la partie pouvant être courbée (6) de la queue creuse (2).

2. Instrument à queue selon la revendication 1, **caractérisé en ce que** la partie pouvant être courbée (6) de la queue creuse (2) est reliée par complémentarité de forme à la partie proximale (4) de la queue creuse (2) et **en ce qu'**un tuyau extérieur (36) s'étendant au moins jusqu'à l'extrémité proximale de la queue creuse (2) entoure radialement la liaison par complémentarité de forme entre la partie pouvant être courbée (6) et la partie proximale (4) de la queue creuse (2), la queue creuse (2) étant axialement mobile par rapport au tuyau extérieur (36).

3. Instrument à queue selon la revendication 1 ou 2, **caractérisé en ce que** l'élément de traction (38) est relié au tuyau extérieur (36) entourant radialement la liaison par complémentarité de forme entre la partie pouvant être courbée (6) et la partie proximale (4) de la queue creuse (2).

4. Instrument à queue selon une des revendications précédentes, **caractérisé en ce que** le manchon (32) et l'élément de traction (38) sont constitués par une pièce commune.

5. Instrument à queue selon une des revendications précédentes, **caractérisé en ce que** la partie pouvant être courbée (6) de la queue creuse (2) est réalisée en un matériau élastique et comprend plusieurs encoches (20, 22) réparties sa longueur et s'étendant dans une direction transversale à son étendue longitudinale.

6. Instrument à queue selon la revendication 5, **caractérisé en ce que** des encoches (20, 22) se suivant directement sont formées dans des zones de la paroi de la partie pouvant être courbée (6) de la queue creuse (2) situées directement à l'opposée l'une de l'autre.

7. Instrument à queue selon l'une des revendications précédentes, **caractérisé en ce que** plusieurs élévations (44), à travers lesquelles passe l'élément de traction (38), sont formées extérieurement à la partie pouvant être courbée (6) de la queue creuse (2).

8. Instrument à queue selon l'une des revendications précédentes, **caractérisé en ce que**, dans la partie distale (8) de la queue creuse (2) est disposé un outil coupant (12) au moins en partie libre, qui est entraîné par un arbre d'entraînement passant par la queue creuse (2).
